# EUROPÄISCHE PATENTANMELDUNG

(11) **EP 0 860 257 A1**
(43) Veröffentlichungstag der Anmeldung: **26.08.1998**
(21) Anmeldenummer: 98102463.1
(22) Anmeldetag: 13.02.1998
(51) Int. Cl.: B28C 7/06, G01N 19/10, G01N 33/38

(54) **Vorrichtung zur Messung der Eigenfeuchte der Bestandteile einer Baustoffmischung**

(30) Priorität: 21.02.1997 DE 19708680; 09.05.1997 DE 19719696; 10.09.1997 DE 19739598
(71) Anmelder: ELBA-WERK Maschinen-Gesellschaft mbH & Co., D-76275 Ettlingen (DE)
(72) Erfinder: Bittmann, Peter, 76571 Gaggenau (DE)
(74) Vertreter: Zahn, Roland, Dipl.-Ing.

(57) **Zusammenfassung**

In Verbindung mit einer Vorrichtung zur Messung der Eigenfeuchte von aus einem Vorratssilo oder dergleichen ausfließenden Schüttstoffen im fließenden Materialstrom mit einem den Materialstrom aufnehmenden, schräg stehenden Leitblech und einer im Leitblech integrierten, den Schüttstoffen entsprechend kalibrierten Feuchtemeßeinrichtung, insbesondere zur Verwendung in Verbindung mit der Messung der Eigenfeuchte der Bestandteile einer Baustoffmischung, wird vorgeschlagen, daß das Leitblech (5) strömungsbeeinflussende Mittel aufweist. Das Leitblech (5) ist dabei dem Vorratssilo (2) unmittelbar benachbart zugeordnet.

## Beschreibung

Die vorliegende Erfindung bezieht sich auf eine Vorrichtung zur Messung der Eigenfeuchte von Schüttstoffen nach dem Oberbegriff des Patentanspruchs 1.

Bei der Herstellung von Baustoffmischungen, insbesondere von Beton, der aus einem erhärtenden Gemisch aus Wasser, Zement, Zu-schlägen wie Sand und Kies, sowie eventuellen weiteren Zusatzmitteln und Zusatzstoffen besteht, ist der richtige Wasser- / Zement-Faktor, d.h. die richtige Dosierung von Wasser und Zement wesentlich, da von dieser die Verarbeitbarkeit des Frischbetons, sowie die Festigkeit des erhärteten Betons abhängt. Weisen die Bestandteile des Betons einer konstanten bekannten Feuchtigkeitsgehalt auf, so ist die Dosierung nicht problematisch. Problematisch wird die Herstellung der Baustoffmischung jedoch dann, wenn der Feuchtigkeitsgehalt der Zuschlage unbekannt ist und wenn er sich übercies noch während der Betriebsdauer ändert Änderungen des Feuchtigkeitsgehalts der Zuschlagstoffe von Charge zu Charge lassen sich wegen der Inhomogenität des Inhalts von Vorratssilos nicht gezielt beeinflussen. Um qualitativ hochwertige Betonmischungen mit jederzeit reproduzierbaren Eigenschaften herzustellen, ist es daher zu Beginn der Herstellung einer jeden Betoncharge erforderlich, den Feuchtigkeitsgehalt der Zuschläge genau zu ermitteln, um die Wasser- und Zuschlagsbilanz entsprechend korrigieren beziehungsweise aktualisieren zu können.

Um den Feuchtigkeitsgehalt der üblicherweise vor ihrer Mischung in Zuschlagsilos bevorrateten Zuschlagsstoffe zu ermitteln, gibt es verschiedene auf der Verwendung von Sonden oder Meßfühlern beruhende Möglichkeiten. Auch wenn die Meßfühler eine ausreichende Meßgenauigkeit aufweiser, so läßt sich häufig noch immer nicht die richtige Eigenfeuchte der Zuschlagssoffe ermitteln, da der Erfassungsbereich der Sonden im Verhältnis zur Größe der zu mischenden homogenen Charge der Zuschlagsstoffe relativ klein ist. Es gelingt so in der Regel nicht, cen repräsentativen Mittelwert der Chargenfeuchte zu bestimmen.

Würde der Meßfühler an der Wandung des Vorratssilos oder in dessen Auslaufbereich oder oberhalb von diesem im noch stehenden Material angeordnet sein, so würden sich wegen der Inhomogenität des Materials und der Schwierigkeit einer hinreichend genauen Mittelwertbildung bei Messungen von oder während der Dosierung große Ungenauigkeiten ergeben.

Würde die Messung mit im Auslaufbereich des Silos angeordneten Meßfühlern ausgeführt werden, so wurden sich Ungenauigkeiten bei der Mittelwertbildung ergeben, da das Schüttgut während des wechselnden Fließvermögens und Fließverhaltens unterschiedliche Dichten aufweist. Bekanntlich ist aber gerade ein reproduzierbarer Dichtewert auch eine Garantie für einen zu einem bestimmten Zu-schlagstoff gehörenden Eigenfeuchte-Wert.

Aus der DE-PS 27 08 943 ist ein Verfahren und eine Vorrichtung zur Messung der Eigenfeuchte von Schüttgütern bekannt, wobei eine Teilmenge einer ausströmenden Schüttgutcharge abgezweigt und verdichtet wird und die Eigenfeuchte dieser Teilmenge anschließend bestimmt wird. Der bekannten Vorrichtung haftet der Nachteil an, daß nur eine relativ kleine Menge der Schüttgutcharge meßtechnisch erfaßt wird.

Aus der DE-PS 35 38 885 ist ein Verfahren und eine Vorrichtung der genannten Art bekannt, bei der während der gesamten Dosierzeit einer Schüttgutcharge ein Teilstrom dieser charge in einem separaten Meßkanal meßtechnisch erfaßt wird. Aus der Mehrzahl der ermittelten Meßwerte wird mittels eines spezifischen Auswerte- beziehungsweise Mittelungsprogramms ein repräsentativer Wert für die Eigenfeuchte der aktuellen Schüttgutcharge gewonnen. Dieser repräsentative Wert repräsentiert jedoch auch hier nur eine Teilmenge.

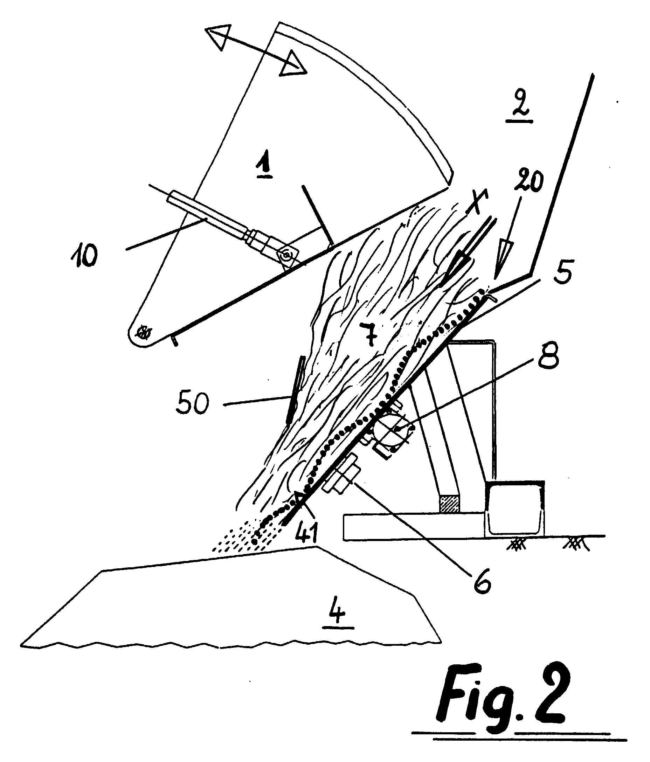

Aus der DE-PS 36 12 282 ist eine Vorrichtung zur Messung des Feuchtigkeitsgehalts von Schüttstoffen bekannt, bei der der gesamte Schüttgutstrom über ein Prallblech geleitet wird, dem ein Feuchtigkeitsfühler zugeordnet ist. Da dieser Meßfühler nur einen begrenzten und relativ geringen Meßbereich erfaßt und nicht davon ausgegangen werden kann, daß die längs des Prallblechs strömende Schüttstoffschicht eine homogene Feuchtigkeitsverteilung aufweist, ist die Meßgenauigkeit dieser Vorrichtung als unzulänglich zu betrachten. Diesbezüglich hat sich darüber hinaus folgendes gezeigt: Der vom Vorratssilo auf das Prallblech aufgefallene Schüttgutstrom bewegt sich nicht homogen beziehungsweise linear über das Prallblech hinweg, sondern in Wellenbewegungen, und zwar aufgrund von Reflexionen des Materialstroms am Prallblech. Daraus resultiert eine völlig unkontrollierte und ungenaue, gegebenenfalls sogar völlig fehlende Berührung zwischen dem Material und der Meßfläche, d.h. dem Prallblech beziehungsweise dem Leitblech, so daß keine reproduzierbaren Meßergebnisse erreichbar sind (vergleiche in Fig. 2 die durch eine Punktlinie angedeutete Welle längs des Leitblechs).

Eine Weiterbildung der vorgenannten Vorrichtung ist Gegenstand der DE-PS 40 01 928. Bei dieser Feuchtemeßeinrichtung wird die von der Inhomogenität der Feuchteverteilung herrührende Unzulänglichkeit der Vorrichtung gemäß der DE-PS 36 12 282 dadurch kompensiert, daß ein Meßkegel vorgesehen ist oder eine Sonde quer zum Schüttstrom bewegt wird.

Desweiteren ist aus der DE 195 12 924 A1 eine Vorrichtung zur Messung der Eigenfeuchte der Bestandteile einer Baustoffmischung bekannt, bei der eine bestimmte Menge des ausfließenden Material mittels einer Kolben-Zylinder-Anordnung gegen das Leitblech gepreßt wird. Auf diese Weise wird jeweils eine definierte Menge des Materialstroms kurzfristig fixiert und bezüglich ihrer Eigenfeuchte "analysiert".

Die der vorliegenden Erfindung zugrunde liegende Aufgabe besteht darin, eine weitere Vorrichtung der gattungsgemäßen Art vorzuschlagen, bei der die Feuchtemeßeinrichtung stets einen repräsentativen Querschnitt des Schüttstoffstroms bei gleichem Dichtewert erfaßt.

Diese Aufgabe wird durch den im kennzeichnenden Teil des Patentanspruchs 1 spezifizierter Aufbau gelöst.

Der Kern dieser Lösung besteht mit anderen als im Anspruch 1 gebrauchten Worten zunächst darin, daß die Schüttgutcharge während der gesamten Ausström- bzw. Dosierzeit so beeinflußt wird, daß bei stets gleichem Dichtewert der akktuelle Feuchtigkeitsgehalt 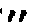 analysiert 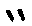 werden kann, wobei - in weiterer Ausgestaltung dieser Grundidee - der aus dem Vorratssilo ausströmende Materialstrom unmittelbar nach dem Ausströmen aus dem Vorratssilo vom Leiblech aufgenommen wird.

Besondere Ausgestaltungen im Hinblick auf die spezifische Art und Weise der Strömungsbeeinflussung und die An- / Zuordnung der Feuchtemeßeinrichtung sind Gegenstand der weiteren Ansprüche.

Der besondere Vorteil der Anordnung der Feuchtemeßeinrichtung am Leitblech besteht darin, daß die Vorrichtung insgesamt relativ kompakt gebaut werden kann und daß Materialablagerungen oder Materialanbackungen am Leitblech verhindert werden, so daß stets eine saubere Meßfläche vornanden ist.

Die Einzelheiten der Erfindung werden im folgenden anhand der Zeichnung näher erläutert, die eine schematische Darstellung eines Dosierverschlusses mit einem Schüttstoffstrom und einer Vorrichtung zur Messung der Eigenfeuchte dieses Schüttstoffs an sich zeigt, wobei
- Fig. 1: das auf einer Stauklappe beruhende Ausführungsbeispiel,
- Fig. 2: die Vorrichtung mit der auf einer Barriere oder dergleichen beruhenden Strömungsbeeinflussung und
- Fig. 3: das Beispiel mit dar schwingend gelagerten Feuchtemeßeinrichtung
zeigen.

In der Zeichnung ist ein Dosierverschluß 1 eines zeichnerisch nicht näher dargestellten Vorratssilos 2 gezeigt, der durch eine hydraulische Kolben-Zylinder-Einheit 10 verschwenkt werden kann, wodurch das Vorratssilo 2 geöffnet und geschlossen werden kann. Unterhalb des Silos 2 befindet sich im allgemeinen ein Behälter 4 zur Aufnahme der einer bestimmten Rezeptur entsprechenden Zuschlagsstoffchargen.

Zwischen dem Silo 2 und dem Behälter 4 ist eine erfindungsgemäße Vorrichtung zur Messung der Eigenfeuchte der Schüttstoff- beziehungsweise Zuschlagstoffchargen plaziert.

Diese Vorrichtung basiert im wesentlichen auf einem Leitblech 5, das unterhalb des Vorratssilos 2 beziehungsweise des Dosierverschlusses 1 angeordnet ist und (im allgemeinen) unmittelbar an einer Ausströmkante 20 des Vorratssilos 2 anschließt. Das Leitblech 5 ist vorzugsweise so an- beziehungsweise zugeordnet, daß der gesamte in einer Dosiercharge ausfließende Materialstrom 7 (vergleiche Pfeil X) über dieses Leitblech 5 fließt.

Dem Leitblech 5 ist an der Unterseite eine Feuchtemeßeinrichtung (Meßsonde) 6 zugeordnet. Diese Feuchtmeßeinrichtung 6 ist den unterschiedlichen Schüttstoffen entsprechend kalibriert, so daß während des Materialflusses über das Leitblech 5 die Eigenfeuchte des aktuellen Schüttstoffs bestimmt werden kann.

Die soweit beschriebene Anordnung gilt für alle dargestellten Ausführungsbeispiele gleichermaßen.

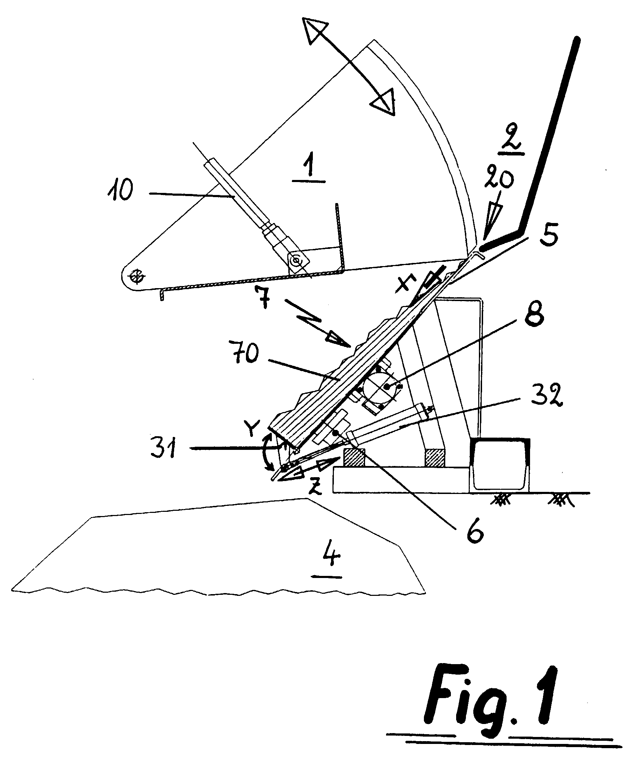

In Fig. 1 ist folgende spezifische Konzeption gezeigt: Am - in Fließrichtung X des Materialstroms 7 betrachtet - Ablaufende des Leitblechs 5 ist eine Stauklappe 31 angeordnet, die relativ zum Leitblech 5 so angestellt werden kann, daß der über das Leitblech 5 fließende Materialstrom 7, 70 aufgestaut wird. Ist die der Höhe der Stauklappe 31 entsprechende Höhe der sich aufstauenden Materialschicht 70 erreicht, so fließt das weitere Material über diese Materialschicht 70 hinweg und bewirkt deren homogene Verdichtung. Damit liegen für die einzelnen Meßvorgänge jeweils gleiche und insbesondere reproduzierbare Dichteverhältnisse vor.

Ist die Messung beendet, wo wird die Stauklappe 31 abgesenkt, so daß die vorher auf dein Leitblech 5 aufgestaute Materialschicht 70 zusammen mit dem weiteren Materialstrom 7 abfließen kann. Das leitblech 5 wird dabei gleichzeitig gereinigt.

Gemäß dem dargestellten Ausführungsbsispiel wird die Stauklappe 31 über einen Zylinder 32 betätigt, der funktional mit der Stauklappe 31 so gekoppelt ist, daß er zwecks Einleitung eines Meßvorgangs die Stauklappe 31 senkrecht zur Fließrichtung X stellt und sie nach Beendigung des Meßvorgangs in die Ebene des Leitblechs 5 absenkt (vergleiche Pfeile Y und Z).

Bei dem in Fig. 2 dargestellten Ausführungsbeispiel geht es primär darum, sicherzustellen, daß der Schüttstoffstrom stets insoweit gleichbleibend über das Leitblech 5 abfließt, daß das Schüttgut nicht in unkontrollierten Wellenbewegungen (vgl. die gepunktete Linie) über das Leitblech 5 abfließt.

Des Schüttgut soll aufgrund einer spezifischen Ausgestaltung der Oberfläche des Leitblechs 5 so geleitetbeziehungsweise beeinflußt werden, daß ein jederzeit reproduzierbares Fließ- und Dichteverhalten und damit reproduzierbere Meßergebnisse erreichbar sind.

In besonderer Ausgestaltung des Leitblechs 5 weist dieses zur Vermeidung unkontrollierbarer Wellenbewegungen beziehungsweise Wellenausbildungen des Materielstroms 7 auf dem (Gleit-) Weg längs des Leitblechs 5 mindestens eine quer zur Fließrichtung X des Materialstroms 7 angeordnete Barriere 41 oder dergleichen auf. Es hat sich gezeigt, daß üher diese Barriere 41 die Wellenausbildung vermieden wird, wodurch eine weitestgehend lineare Strömung des Materials über die Feuchtemeßeinrichung 6 erreicht wird, was zu ganz erheblich besseren und insbesondere reproduzierbaren Meßergebnissen führt, und zwar selbst dann, wenn der Materialstrom 7 - analog zum Konzept gemäß der DE-PS 36 12 282 - im freien Fall auf das Leitblech (Prallblech) fällt.

Zusätzlich zu der (den) quer zur Fließrichtung X angeordneten Barriere(n) können auf dem leitblech 5 auch weitere, insbesondere schräg zur Fließrichtung X angeordnete Barrieren vorgesehen werden.

Grundsätzlich ist es auch möglich und gegebenenfalls sinnvoll, dem Leitblech 5, insbesondere im Bereich der Feuchtemeßeinrichtung 6, ein zweites Leitblech 50 zuzuordnen. Dieses zweite Leitblech 50 bildet gemeinsam mit dem Leitblech 5 einen konischen Kanal, so daß die Verdichtungswirkung auf den Materialstroms 7 erhöht wird.

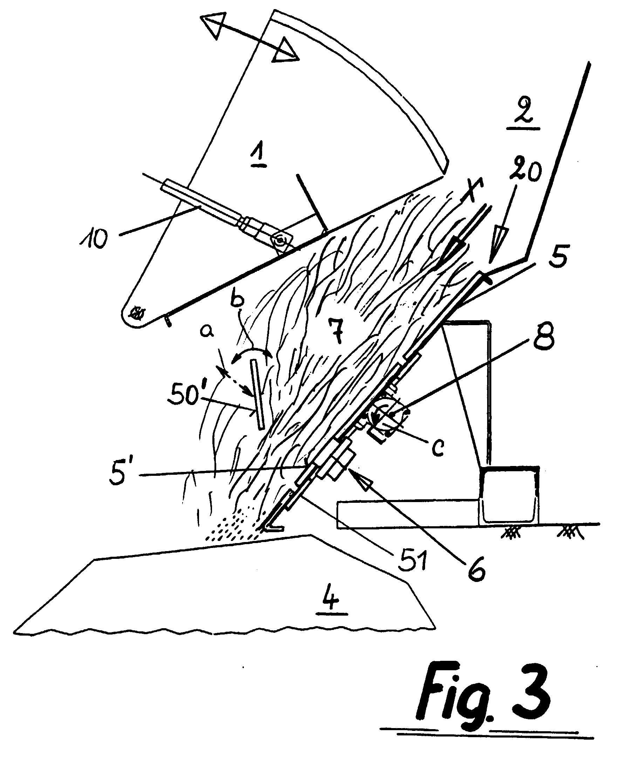

Bei dem in Fig. 3 dargestellten Ausführungsbeispiel wird insoweit von Fig. 2 ausgegangen, als dafür Sorge getragen werden soll, daß das Schüttgut nicht in unkontrollierten Wellenbewegungen über das Leitblech 5 abfließt. Dies wird dadurch erreicht, daß das Schüttgut aufgrund einer flexiblen Aufhängung der Feuchtemeßeinrichtung 6, d.h. aufgrund der spezifischen Gestaltung des Leitblechs 5 stets über die Meßfläche der Feuchtemeßeinrichtung 6 geleitet wird. Es liegt mithin ein stets reproduzierbares Fließ- und Dichteverhalten vor, womit jederzeit reproduzierbare Meßergebnisse gewährleistet sind, zumal auch die Selbstreinigung der Meßfläche ganz erheblich verbessert ist.

In besonderer Ausgestaltung des Leitblechs 5 ist dieses - in Fließrichtung X des Materialstroms 7 betrachtet - etwa mittig ausgeschnitten (vgl. 5'). Dieser Ausschnitt ist seinerseits wieder mit Hilfe eines komplementären Plattenzuschnitts 5' geschlossen, der einerseits als integrierten Bestandteil die Feuchtemeßsinrichtung 6 trägt und der andererseits über eine den Rand des Ausschnitts im Leitblech 5 und dem Außenrand des Plattenzuschnitts 5' verbindende Gummiplatte 51 am Leitblech 5 fixiert ist.

Es hat sich gezeigt, daß aufgrund dieser flexiblen Aufhängung der Feuchtemeßeinrichtung 6 die Wellenausbildung vermieden ist, wodurch eine weitestgehend lineare homogene Strömung des Materialstroms 7 über die Feuchtemeßeinrichtung 6 erreicht wird, was zu ganz erheblich besseren und insbesondere reproduzierbaren Meßergebnissen führt, und zwar unabhängig davon, ob der Materialstrom im freien Fell auf das Leitblech 5 fällt oder ob das Leitblech 5 unmittelbar an die Ausströmkante 20 des Vorratssilos 2 anschließt.

In weiterer Ausgestaltung der Feuchtemeß-Vorrichtung ist vorgesehen, dem Leitblech 5, urd zwar insbesondere im Bereich der Feuchtemeßeinrichtung 6, ein zweites Leitblech 50' zuzuordnen. Dieses zweite Leitblech 50 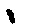 bildet somit gemeinsam mit dem Leitblech 5 einen konischen Kanal, so daß die Verdichtungswirkung auf den durchfließenden Teilstrom des Materials bzw. auf den Materialstrom 7 als Ganzem erhöht wird. Zur Optimierung dieser Kanal- bzw. Verdichtungswirkung ist dieses zweite Leitblech 50 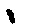 höhen- und winkelmäßig (vgl. Pfeile a und b) verstellbar konzipiert, so daß der Spalt zwischen dem Leitblech 5 und dem zweiten Leitblech 50 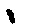 jeweils dem spezifischen Material entsprechend eingestellt werden kann.

Zur weiteren Unterstützung des Verdichtungseffekts kann dem Leitblech 5 von der Unterseite her zusätzlich ein Rüttler 8 zugeordnet werden. Dieser Rüttler 8 ist gleichermaßen wie die Feuchtemeßeinrichtung 6 am Plattenzuschritt 5 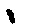 fixiert und er ist darüberhinaus so konzipiert, daß er in Fließrichtung X wirkende Schwingungen ausführt (vgl. Pfeil c). Der Rüttler 8 unterstützt mithin das Fließverhalten des Materialstroms 7 und kann selbstverständlich auch am Leitblech 5 entsprechend den Ausführungsbeispielen nach Fig. 1 und Fig. 2 fixiert werden.

## Patentansprüche

1. Vorrichtung zur Messung der Eigenfeuchte von aus einem Vorratssilo oder dergleichen ausfließenden Schüttstoffen im fließenden Materialstrom mit einem den Materialstromsaufnehmenden, schräg stehenden Leitblech und einer im Leitblech integrierten, den Schüttstoffen entsprechend kalibrierten Feuchtemeßeinrichtung, insbesondere zur Verwendung in Verbindung mit der Messung der Eigenfeuchte der Bestandteile einer Baustoffmischung,
dadurch gekennzeichnet,
daß das Leitblech (5) strömungsbeeinflussende Mittel aufweist.

2. Vorrichtung nach Anspruch 1,
dadurch gekennzeichnet,
daß das Leitblech (5) dem Vorretssilo (2) unmittelbar benachbart zugeordnet ist.

3. Vorrichtung nach Anspruch 1 oder 2,
dadurch gekennzeichnet,
daß dem Leitblech (5) in Fließrichtung (X) des Materialstroms (7) betrachtet am Ablaufende eine insbesondere über einen Zylinder (32) betätigbare Stauklappe (31) zugeordnet ist, die bei über des Leitblech (5) fließendem Materialstrom (7) dem Leitblech (5) gegenüber hochgestellt wird, um das Material längs des Leitblechs (5) aufzustauen, und
die nach Beendigung des Meßvorgangs abgesenkt wird, so daß die aufgestaute Materialschicht (70) abfließen kann.

4. Vorrichtung nach Anspruch 1 oder 2,
dadurch gekennzeichnet,
daß auf der Oberfläche des Leitblechs (5) mindestens eine quer zur Fließrichtung (X) angeordnete Barriere (41) oder ein vergleichbares strömungsbeeinflussendes Hindernis vorgesehen ist.

5. Vorrichtung nach Anspruch 4,
dadurch gekennzeichnet,
daß dem Leitblech (5) ein zweites insbesondere höhen- und winkelverstellberes Leitblech (50, 50 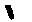 ) gegenüberliegt und der Materialstrom zwischen diesen beiden Leitblechen (5; 50, 50 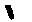 ) hindurchfließt.

6. Vorrichtung nach einem der Ansprüche 4 oder 5,
dadurch gekennzeichnet,
daß zusätzlich mindestens eine schräg zur Fließrichtung angeordnete Barriere oder dergleichen vorgesehen sind.

7. Vorrichtung nach einem der Ansprüche 1 bis 6,
dadurch gekennzeichnet,
daß die Feuchtemeßeinrichtung (6) in Fließrichtung (X) des Materialstroms (7) betrachtet dem Ablaufende des Leitblechs (5) benachbart angeordnet ist.

8. Vorrichtung nach Anspruch 7,
dadurch gekennzeichnet,
daß die Feuchtemeßeinrichtung (6) relativ zum Leitblech (5) elastisch gelagert ist.

9. Vorrichtung nach Anspruch 8,
dadurch gekennzeichnet,
daß die Feuchtemeßeinrichtung (6) am Leitblech (5) an einem zu einem Ausschnitt im Leitblech (5) komplementären Plattenzuschnitt (5 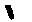 ) fixiert und der Plattenzuschnitt (5 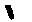 ) relativ zum Leitblech (5) über eine elastische Matte, beispielsweise eine Gummiplatte (51), aufgehängt ist.

10. Vorrichtung nach einem der Ansprüche 1 bis 9,
dadurch gekennzeichnet,
daß dem Leitblech (5) beziehungsweise dem Plattenzuschnitt (5 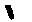 ) ein Rüttler (8) zugeordnet ist.
